# EUROPEAN PATENT APPLICATION

(11) **EP 1 681 358 A1**
(43) Date of publication of application: **19.07.2006**
(21) Application number: 06250261.2
(22) Date of filing: 18.01.2006
(51) Int. Cl.: C12Q 1/68

(54) **Identification of rare alleles by enzymatic enrichment of mismatched heteroduplexes**

(30) Priority: 18.01.2005 US 39585
(71) Applicant: Affymetrix, Inc., Santa Clara, CA 95051 (US)
(72) Inventor: Fahkrai-Rad, Hossein, Sunnyvale, California 94085 (US); Eberle, James, Burlingame, California 94010 (US); Zheng, Jianbiao, Fremont, California 94555 (US); Willis, Thomas, San Francisco, California 94133 (US); Wong, Kee, San Francisco, California 94134 (US); Suyenaga, Kent, South San Francisco, California 94083 (US); Moorhead, Martin, San Mateo, California 94402 (US)
(74) Representative: Bizley, Richard Edward

(57) **Abstract**

In one aspect, the invention provides a method of enriching a pool of nucleic acid duplexes for heteroduplexes containing sequences that vary from those of a reference DNA population. A mixture of test sequences, that is, nucleic acids suspected of containing one or more mutations or rare alleles, and corresponding reference sequences are denatured and reassociated so that duplexes form comprising heteroduplexes and homoduplexes. In accordance with one aspect of the invention, whenever a subset of heteroduplexes contains mismatched sequences, the mismatches are corrected so that the sequences of strands from the test population are preserved, or selected. The preserved or selected sequences may then be denatured and reassociated with additional sequences from the reference population to once again form duplexes comprising homoduplexes and heteroduplexes, thereby generating an enriched population of variant sequences.

## Description

### Field of the Invention

The invention relates generally to a method of discovering mutations or rare alleles in a population of individuals, and more particularly, to a method of enriching such a population for mutations or rare alleles for detection by sequence analysis.

### BACKGROUND

Random sequencing approaches have led to the identification of a tremendous number of single nucleotide polymorphisms (SNPs) in the human genome. Through the work of The SNP Consortium, 1.4 million SNPs have been identified, e.g. Holden, pgs. 22-26, Biotechniques, Supplement (June, 2002). This has been followed by other public projects leading to the presence of about 3 million SNPs with some level of validation. These SNPs provide researchers with a wealth of candidate SNPs in their desired candidate regions. Unfortunately, only a fraction of the disease-causing variations in regulatory and coding regions (cSNP) are identified through this approach, e.g. Kruglyak and Nickerson, Nature Genetics, 27: 234-236 (2001); Carlson et al, Nature Genetics, 33: 518-521 (2003). An extensive targeted effort to identify common cSNPs has been advocated (Johnson et al, Nature Genetics, 29: 233-237 (2001)) and partially implemented (Haga et al, J. Hum. Genet., 47: 605-610 (2002)), but cost has been a major drawback for such an approach. In order to have 95% confidence of identifying an allele with a 2% frequency, 75 individuals need to be sequenced due to the Poisson statistics of chromosome sampling. In addition, detection of these alleles assumes the ability to detect heterozygote peaks in a sequencing trace with good accuracy. Unfortunately, conventional sequencing approaches do not have sufficient sensitivity to detect reliably rare alleles or mutant sequences in a pool of sequences when their abundances are less than a few percent, e.g. Blazej et al, Genome Research, 13: 287-293 (2003).

In view of the above, the availability of methods that permit the identification of rare alleles and/or mutations in pools, or mixed populations, of nucleic acid sequences, such as a pool of genomic sequences, would be advantageous in several fields, including genetics research, medical genetics, and oncology.

### SUMMARY OF THE INVENTION

The invention provides a method for identifying rare alleles and/or mutations in a pool of nucleic acids from a selected genetic locus. In one aspect, a method of the invention permits the identification of rare alleles in a pool of nucleic acids from the same genetic locus from a plurality of individuals. In another aspect, a method of the invention permits the identification of mutations in a pool of nucleic acids extracted from a tumor sample of a cancer patient, wherein such nucleic acids are from the same genetic locus.

In one embodiment, a method of the invention comprises the following steps: (i) forming duplexes between test sequences and reference sequences thereof such that heteroduplexes containing one or more mismatches form whenever a rare allele is present in a test sequence, and such that the test sequences initially are sequences from the genetic locus of individuals of the population; (ii) enriching the amount of heteroduplexes containing such one or more mismatches by treating the duplexes with enzymes having mismatch-recognition activity, nucleic acid polymerase activity, and exonuclease activity, the mismatch-recognition activity binding to each of the one or more mismatches, the exonuclease activity digesting one or more portions of each heteroduplex containing such one or more mismatches, and the nucleic acid polymerase activity replacing such digested portions whenever a heteroduplex contains at least one mismatch between a test sequence and a reference sequence; (iii) repeating steps (i) and (ii) until at least five percent of the duplexes are heteroduplexes containing one or more mismatches or until steps (i) and (ii) have been performed a predetermined number of times; and (iv) sequencing at least one strand of the duplexes to form a sequence of base-call signals, the base-call signals giving the identity and quantity of nucleotides at each sequence position of a strand of the duplex.

The invention advances the art of rare allele and mutation detection in pooled samples by providing a method of enriching samples for non-reference sequences in order to overcome the lack of sensitivity of current sequencing approaches for detecting sequence variants that make up less than about five percent of a pooled sample.

### Brief Description of the Drawings

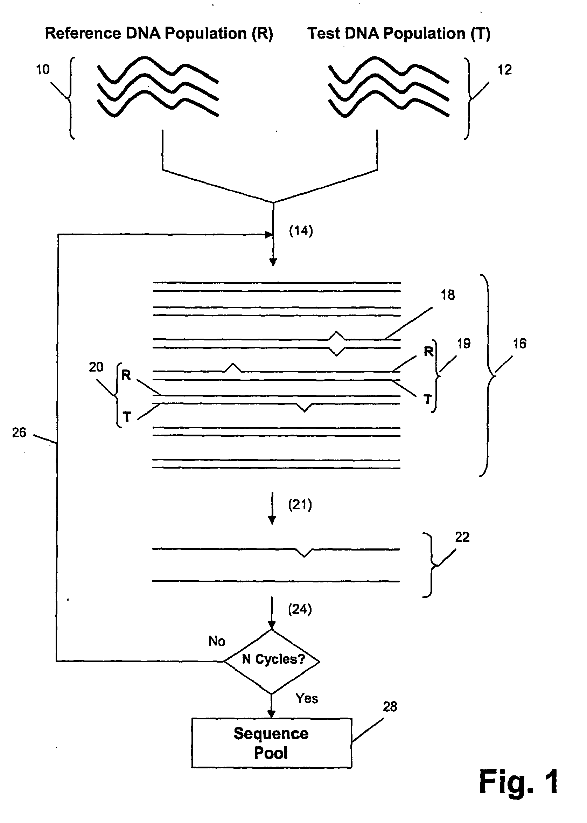

Fig. 1 diagrammatically illustrates the general concept of the method of the invention.

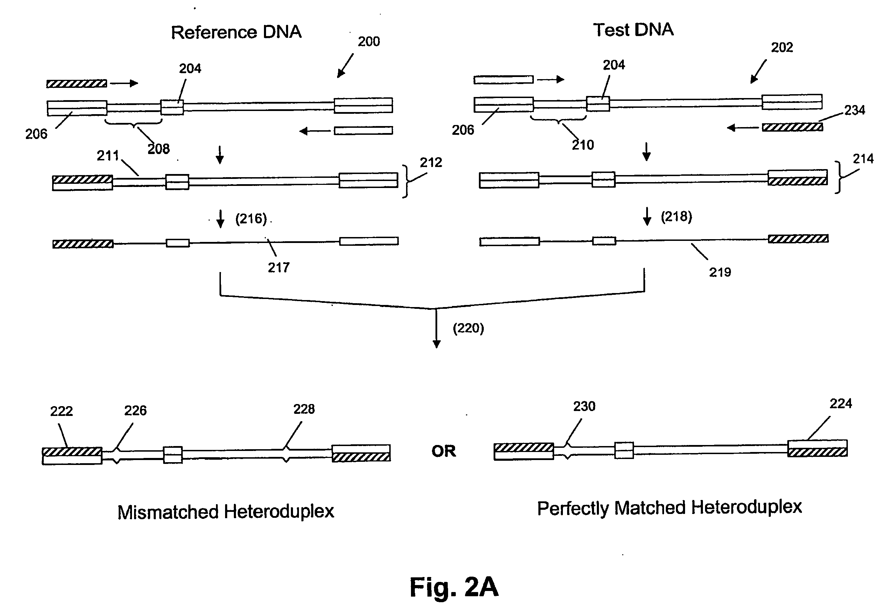

Figs. 2A-2C diagrammatically illustrates steps for in vitro selection of a test strand forming a mismatch with its corresponding reference strand.

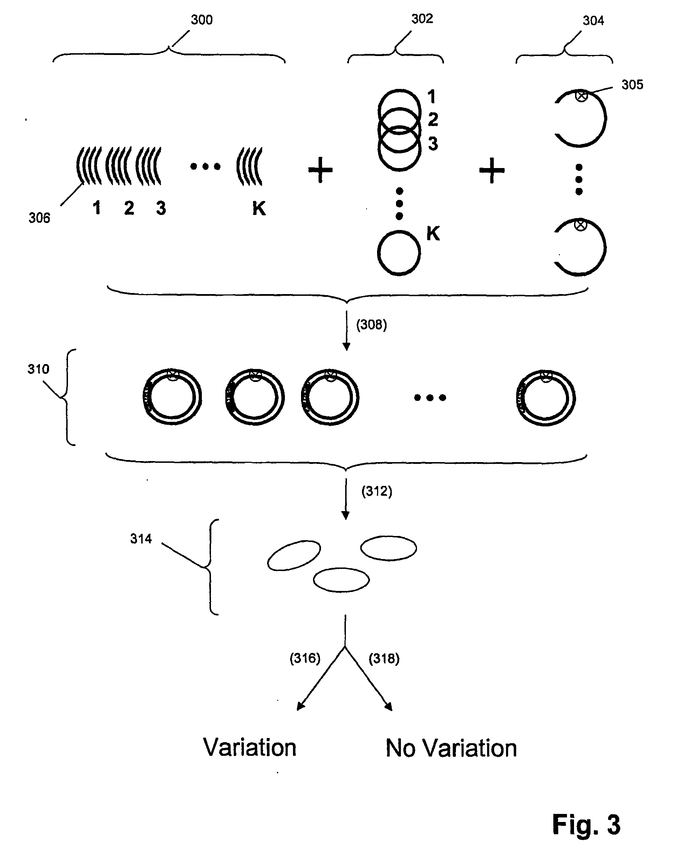

Fig. 3 diagrammatically illustrates steps of the mismatch repair detection (MRD) process.

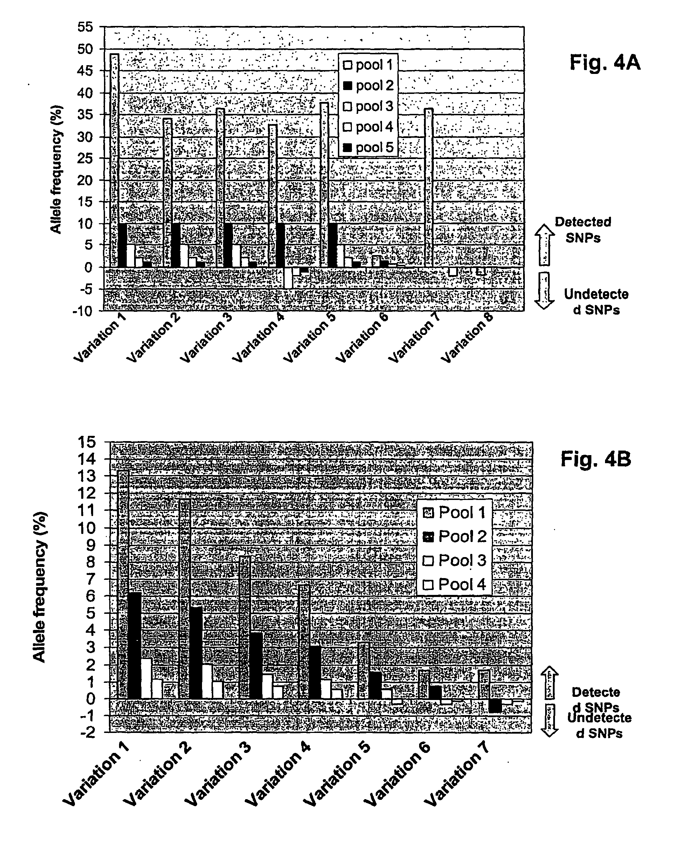

Fig. 4A. Detection of known SNPs. This figure depicts the allele frequency (Y axis) of the 7 detected variations in the four constructed genomic mixtures (X axis) with data from each pool represented with a different color. Pool 1 is where all 94 samples are mixed. The other 4 pools are different mole DNA dilutions of the 5-genome pool. To distinguish SNPs that were detected from those that were not detected in a particular genomic mixture in the MRD based SNP discovery we depict the undetected SNPs with negative allele frequency. Variation 7 and 8 are in the same amplicon. Sequence data for this amplicon was obtained for only two pools. Data from pool 1 of this amplicon demonstrates how the presence of a common variant (variation 7) masks other rare variant(s) (variant 8) in the same amplicon.
Fig. 4B. Detection of unknown SNPs. This figure depicts the allele frequency (Y axis) of the 7 detected variations in the four constructed genomic mixtures (X axis) with data from each pool represented with a different color. The 4 pools shown are for the different mole DNA dilutions of the 5-genome pool. To distinguish SNPs that were detected from those that were not detected in a particular genomic mixture in the MRD based SNP discovery we depict the undetected SNPs with negative allele frequency. Some variations are detected when present at frequency as low as 0.5%.

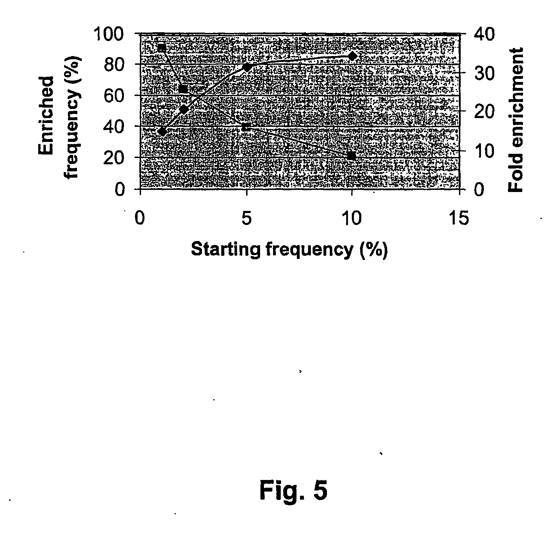

Fig. 5 shows the average enrichment for detected SNPs with starting allele frequencies of 1, 2, 5 and 10%. To generate this graph, we obtained the average frequency in the variant pool of the detected SNPs that were initially at 1, 2, 5 or 10%. The estimation of the enriched frequency was done as previously described, Kwok et al (1994), Genomics, 23: 138-144. As expected the rarer alleles have higher fold enrichment but continue to have lower frequency in the enriched pool. For example 1% and 10% alleles get enriched 36 and 8.5 folds respectively to achieve in the variant pool 36% and 85% frequencies, respectively.

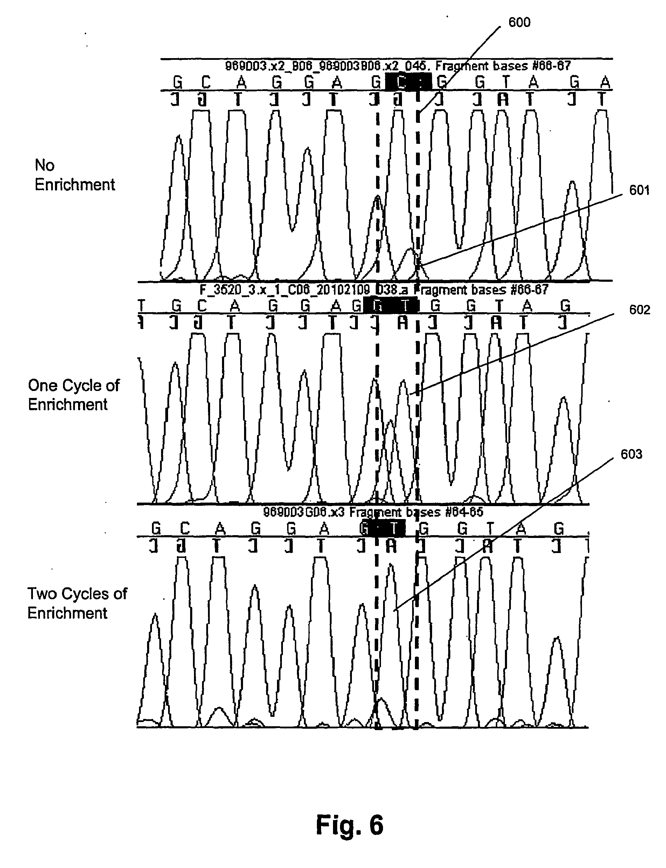

Fig. 6 shows sequencing traces of products that had undergone 0 (A), 1 (B) or 2 (C) MRD rounds. Box (600) indicates the site of variation (C/T SNP). The reference has the C and the variant base is the T. The top trace is a sequence of the PCR amplified from 100 Caucasians. The T/C polymorphism is evident after the first round of enrichment and after the second round the T becomes the only major peak (603).

### Definitions

Terms and symbols of nucleic acid chemistry, biochemistry, genetics, and molecular biology used herein follow those of standard treatises and texts in the field, e.g. Kornberg and Baker, DNA Replication, Second Edition (W.H. Freeman, New York, 1992); Lehninger, Biochemistry, Second Edition (Worth Publishers, New York, 1975); Strachan and Read, Human Molecular Genetics, Second Edition (Wiley-Liss, New York, 1999); Eckstein, editor, Oligonucleotides and Analogs: A Practical Approach (Oxford University Press, New York, 1991); Gait, editor, Oligonucleotide Synthesis: A Practical Approach (IRL Press, Oxford, 1984); and the like.

"Addressable" or "addressed" in reference to tag complements means that the nucleotide sequence, or perhaps other physical or chemical characteristics, of a tag complement can be determined from its address, i.e. a one-to-one correspondence between the sequence or other property of the tag complement and a spatial location on, or characteristic of, the solid phase support to which it is attached. Preferably, an address of a tag complement is a spatial location, e.g. the planar coordinates of a particular region containing copies of the tag complement. However, tag complements may be addressed in other ways too, e.g. by microparticle size, shape, color, signal of micro-transponder, or the like, e.g. Chandler et al, PCT publication WO 97/14028.

"Allele frequency" in reference to a genetic locus means the frequency of occurrence within a population of a particular nucleotide or sequence segment, or other sequence marker, such as an insertion or deletion of one or more nucleotides, or a particular sequence motif, at or within the genetic locus. In reference to the above, a population may be a population of individual from a defmed group, e.g. Caucasian women over the age of 50, or a population may be a population of cells from an individual suffering from a disease or condition, such as cancer. In some contexts, an allele frequency may also refer to the frequency of sequences not identical to, or exactly complementary to, a reference sequence, or a set of reference sequences.

"Amplicon" means the product of an amplification reaction. That is, it is a population of polynucleotides, usually double stranded, that are replicated from one or more starting sequences. The one or more starting sequences may be one or more copies of the same sequence, or it may be a mixture of different sequences. Amplicons may be produced in a polymerase chain reaction (PCR), by replication in a cloning vector, by linear amplification by an RNA polymerase, such as T7 or SP6, by rolling circle amplification, e.g. Lizardi, U.S. patent 5,854,033 or Aono et al, Japanese patent publ. JP 4-262799; by whole-genome amplification schemes, e.g. Hosono et al, Genome Research, 13: 959-969 (2003), or by like techniques.

"Complement" or "tag complement" as used herein in reference to oligonucleotide tags refers to an oligonucleotide to which an oligonucleotide tag specifically hybridizes to form a perfectly matched duplex or triplex. In embodiments where specific hybridization results in a triplex, the oligonucleotide tag may be selected to be either double stranded or single stranded. Thus, where triplexes are formed, the term "complement" is meant to encompass either a double stranded complement of a single stranded oligonucleotide tag or a single stranded complement of a double stranded oligonucleotide tag.

"Duplex" means at least two oligonucleotides and/or polynucleotides that are fully or partially complementary undergo Watson-Crick type base pairing among all or most of their nucleotides so that a stable complex is formed. The terms "annealing" and "hybridization" are used interchangeably to mean the formation of a stable duplex. "Perfectly matched" in reference to a duplex means that the poly- or oligonucleotide strands making up the duplex form a double stranded structure with one another such that every nucleotide in each strand undergoes Watson-Crick basepairing with a nucleotide in the other strand. The term "duplex" comprehends the pairing of nucleoside analogs, such as deoxyinosine, nucleosides with 2-aminopurine bases, PNAs, and the like, that may be employed. A "mismatch" in a duplex between two oligonucleotides or polynucleotides means that a pair of nucleotides in the duplex fails to undergo Watson-Crick bonding.

"Genetic locus," or "locus" in reference to a genome or target polynucleotide, means a contiguous subregion or segment of the genome or target polynucleotide. As used herein, genetic locus, or locus, may refer to the position of a gene or portion of a gene in a genome, or it may refer to any contiguous portion of genomic sequence whether or not it is within, or associated with, a gene. Preferably, a genetic locus refers to any portion of genomic sequence from a few tens of nucleotides, e.g. 10-30, or 10-100, in length, to a few hundred nucleotides, e.g. 100-1000 or 100-500 in length, to a few thousands of nucleotide in length, e.g. 1000-10,000 or 1000-3000 in length. In some contexts, genetic locus may refer to the location of a nucleotide within a genome.

"Kit" as used herein refers to any delivery system for delivering materials. In the context of reaction assays, such delivery systems include systems that allow for the storage, transport, or delivery of reaction reagents (e.g., probes, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. Such contents may be delivered to the intended recipient together or separately. For example, a first container may contain an enzyme for use in an assay, while a second container contains probes.

"Ligation" means to form a covalent bond or linkage between the termini of two or more nucleic acids, e.g. oligonucleotides and/or polynucleotides, in a template-driven reaction. The nature of the bond or linkage may vary widely and the ligation may be carried out enzymatically or chemically. As used herein, ligations are usually carried out enzymatically to form a phosphodiester linkage between a 5' carbon of a terminal nucleotide of one oligonucleotide with 3' carbon of another oligonucleotide. A variety of template-driven ligation reactions are described in the following references, which are incorporated by reference: Whitely et al, U.S. patent 4,883,750; Letsinger et al, U.S. patent 5,476,930; Fung et al, U.S. patent 5,593,826; Kool, U.S. patent 5,426,180; Landegren et al, U.S. patent 5,871,921; Xu and Kool, Nucleic Acids Research, 27: 875-881 (1999); Higgins et al, Methods in Enzymology, 68: 50-71 (1979); Engler et al, The Enzymes, 15; 3-29 (1982); and Namsaraev, U.S. patent publication 2004/0110213.

"Microarray" refers to a solid phase support that carries oligo- or polynucleotides fixed or immobilized, usually covalently, at specific addressable locations. Preferably, a microarray is a solid phase support having a planar surface, which carries an array or grid of nucleic acids, each member of the array comprising identical copies of an oligonucleotide or polynucleotide immobilized to a fixed region, which does not overlap with those of other members of the array. Typically, the oligonucleotides or polynucleotides are single stranded and are covalently attached to the solid phase support at known, determinable, or addressable, locations. The density of non-overlapping regions containing nucleic acids in a microarray is typically greater than 100 per cm², and more preferably, greater than 1000 per cm². Microarray technology is reviewed in the following references: Schena, Editor, Microarrays: A Practical Approach (IRL Press, Oxford, 2000); Southern, Current Opin. Chem. Biol., 2:404-410 (1998); Nature Genetics Supplement, 21: 1-60 (1999).

"Mismatch" means a base pair between any two of the bases A, T (or U for RNA), G, and C other than the Watson-Crick base pairs G-C and A-T. The eight possible mismatches are A-A, T-T, G-G, C-C, T-G, C-A, T-C, and A-G.

"Mutation" and "polymorphism" are usually used somewhat interchangeably to mean a DNA molecule, such as a gene, that differs in nucleotide sequence from a reference DNA sequence, or wild type sequence, or normal tissue sequence, by one or more bases, insertions, and/or deletions. In some contexts, the usage of Cotton (Mutation Detection, Oxford University Press, Oxford, 1997) is followed in that a mutation is understood to be any base change whether pathological to an organism or not, whereas a polymorphism is usually understood to be a base change with no direct pathological consequences.

"Nucleoside" as used herein includes the natural nucleosides, including 2'-deoxy and 2'-hydroxyl forms, e.g. as described in Kornberg and Baker, DNA Replication, 2nd Ed. (Freeman, San Francisco, 1992). "Analogs" in reference to nucleosides includes synthetic nucleosides having modified base moieties and/or modified sugar moieties, e.g. described by Scheit, Nucleotide Analogs (John Wiley, New York, 1980); Uhlman and Peyman, Chemical Reviews, 90: 543-584 (1990), or the like, with the proviso that they are capable of specific hybridization. Such analogs include synthetic nucleosides designed to enhance binding properties, reduce complexity, increase specificity, and the like. Polynucleotides comprising analogs with enhanced hybridization or nuclease resistance properties are described in Uhlman and Peyman (cited above); Crooke et al, Exp. Opin. Ther. Patents, 6: 855-870 (1996); Mesmaeker et al, Current Opinion in Structual Biology, 5: 343-355 (1995); and the like. Exemplary types of polynucleotides that are capable of enhancing duplex stability include oligonucleotide N3'→P5' phosphoramidates (referred to herein as "amidates"), peptide nucleic acids (referred to herein as "PNAs"), oligo-2'-O-alkylribonucleotides, polynucleotides containing C-5 propynylpyrimidines, locked nucleic acids (LNAs), and like compounds. Such oligonucleotides are either available commercially or may be synthesized using methods described in the literature.

"Perfectly matched" in reference to a duplex means that the poly- or oligonucleotide strands making up the duplex form a double stranded structure with one other such that every nucleotide in each strand undergoes Watson-Crick basepairing with a nucleotide in the other strand. The term also comprehends the pairing of nucleoside analogs, such as deoxyinosine, nucleosides with 2-aminopurine bases, and the like, that may be employed. In reference to a triplex, the term means that the triplex consists of a perfectly matched duplex and a third strand in which every nucleotide undergoes Hoogsteen or reverse Hoogsteen association with a basepair of the perfectly matched duplex. Conversely, a "mismatch" in a duplex between a tag and an oligonucleotide means that a pair or triplet of nucleotides in the duplex or triplex fails to undergo Watson-Crick and/or Hoogsteen and/or reverse Hoogsteen bonding.

"Polynucleotide" or "oligonucleotide" are used interchangeably and each mean a linear polymer of nucleotide monomers. Monomers making up polynucleotides and oligonucleotides are capable of specifically binding to a natural polynucleotide by way of a regular pattern of monomer-to-monomer interactions, such as Watson-Crick type of base pairing, base stacking, Hoogsteen or reverse Hoogsteen types of base pairing, or the like. Such monomers and their internucleosidic linkages may be naturally occurring or may be analogs thereof, e.g. naturally occurring or non-naturally occurring analogs. Non-naturally occurring analogs may include PNAs, phosphorothioate internucleosidic linkages, bases containing linking groups permitting the attachment of labels, such as fluorophores, or haptens, and the like. Whenever the use of an oligonucleotide or polynucleotide requires enzymatic processing, such as extension by a polymerase, ligation by a ligase, or the like, one of ordinary skill would understand that oligonucleotides or polynucleotides in those instances would not contain certain analogs of internucleosidic linkages, sugar moities, or bases at any or some positions. Polynucleotides typically range in size from a few monomeric units, e.g. 5-40, when they are usually referred to as "oligonucleotides," to several thousand monomeric units. Whenever a polynucleotide or oligonucleotide is represented by a sequence of letters (upper or lower case), such as "ATGCCTG," it will be understood that the nucleotides are in 5'→3' order from left to right and that "A" denotes deoxyadenosine, "C" denotes deoxycytidine, "G" denotes deoxyguanosine, and "T" denotes thymidine, "I" denotes deoxyinosine, "U" denotes uridine, unless otherwise indicated or obvious from context. Unless otherwise noted the terminology and atom numbering conventions will follow those disclosed in Strachan and Read, Human Molecular Genetics 2 (Wiley-Liss, New York, 1999). Usually polynucleotides comprise the four natural nucleosides (e.g. deoxyadenosine, deoxycytidine, deoxyguanosine, deoxythymidine for DNA or their ribose counterparts for RNA) linked by phosphodiester linkages; however, they may also comprise non-natural nucleotide analogs, e.g. including modified bases, sugars, or internucleosidic linkages. It is clear to those skilled in the art that where an enzyme has specific oligonucleotide or polynucleotide substrate requirements for activity, e.g. single stranded DNA, RNA/DNA duplex, or the like, then selection of appropriate composition for the oligonucleotide or polynucleotide substrates is well within the knowledge of one of ordinary skill, especially with guidance from treatises, such as Sambrook et al, Molecular Cloning, Second Edition (Cold Spring Harbor Laboratory, New York, 1989), and like references.

"Primer" means an oligonucleotide, either natural or synthetic that is capable, upon forming a duplex with a polynucleotide template, of acting as a point of initiation of nucleic acid synthesis and being extended from its 3' end along the template so that an extended duplex is formed. Extension of a primer is usually carried out with a nucleic acid polymerase, such as a DNA or RNA polymerase. The sequence of nucleotides added in the extension process is determined by the sequence of the template polynucleotide. Usually primers are extended by a DNA polymerase. Primers usually have a length in the range of from 14 to 40 nucleotides, or in the range of from 18 to 36 nucleotides. Primers are employed in a variety of nucleic amplification reactions, for example, linear amplification reactions using a single primer, or polymerase chain reactions, employing two or more primers. Guidance for selecting the lengths and sequences of primers for particular applications is well known to those of ordinary skill in the art, as evidenced by the following references that are incorporated by reference: Dieffenbach, editor, PCR Primer: A Laboratory Manual, 2^{nd} Edition (Cold Spring Harbor Press, New York, 2003).

"Readout" means a parameter, or parameters, which are measured and/or detected that can be converted to a number or value. In some contexts, readout may refer to an actual numerical representation of such collected or recorded data. For example, a readout of fluorescent intensity signals from a microarray is the address and fluorescence intensity of a signal being generated at each hybridization site of the microarray; thus, such a readout may be registered or stored in various ways, for example, as an image of the microarray, as a table of numbers, or the like.

"Reference population" of DNA, or "reference DNA population," refers to a collection of DNAs, or RNAs derived from it, which is compared to a test population of DNA or RNA, (or "test DNA population") by the formation ofheteroduplexes between the complementary strands of the reference DNA population and test DNA population. If perfectly matched heteroduplexes form, then the respective members of the reference and test populations are identical; otherwise, they are variants of one another. Typically, the nucleotide sequences of members of the reference population are known and the sequences typically are listed in sequence databases, such as Genbank, Embl, or the like. A reference population of DNA comprises a cDNA library from a known cell type or tissue source. For example, a reference population of DNA may comprise a cDNA library derived from the tissue of a healthy individual and a test population of DNA may comprise a cDNA library derived from the same tissue of a diseased individual. Alternately, a reference DNA population may comprise genomic DNA from normal tissue of a cancer patient and a test DNA population may comprise DNA from a tumor of the same patient Reference populations of DNA may also comprise an assembled collection of individual polynucleotides, cDNAs, genes, or exons thereof, e.g. genes or exons encoding all or a subset of known p53 variants, or the like.

"Sample" means a quantity of material from a biological, environmental, medical, or patient source in which detection or measurement of target nucleic acids is sought. On the one hand it is meant to include a specimen or culture (e.g., microbiological cultures). On the other hand, it is meant to include both biological and environmental samples. A sample may include a specimen of synthetic origin. Biological samples may be animal, including human, fluid, solid (e.g., stool) or tissue, as well as liquid and solid food and feed products and ingredients such as dairy items, vegetables, meat and meat by-products, and waste. Biological samples may include materials taken from a patient including, but not limited to cultures, blood, saliva, cerebral spinal fluid, pleural fluid, milk, lymph, sputum, semen, needle aspirates, and the like. Biological samples may be obtained from all of the various families of domestic animals, as well as feral or wild animals, including, but not limited to, such animals as ungulates, bear, fish, rodents, etc. Environmental samples include environmental material such as surface matter, soil, water and industrial samples, as well as samples obtained from food and dairy processing instruments, apparatus, equipment, utensils, disposable and non-disposable items. These examples are not to be construed as limiting the sample types applicable to the present invention.

"Solid support", "support", and "solid phase support" are used interchangeably and refer to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations. Microarrays usually comprise at least one planar solid phase support, such as a glass microscope slide.

"Specific" or "specificity" in reference to the binding of one molecule to another molecule, such as a labeled target sequence for a probe, means the recognition, contact, and formation of a stable complex between the two molecules, together with substantially less recognition, contact, or complex formation of that molecule with other molecules. In one aspect, "specific" in reference to the binding of a first molecule to a second molecule means that to the extent the first molecule recognizes and forms a complex with another molecules in a reaction or sample, it forms the largest number of the complexes with the second molecule. Preferably, this largest number is at least fifty percent. Generally, molecules involved in a specific binding event have areas on their surfaces or in cavities giving rise to specific recognition between the molecules binding to each other. Examples of specific binding include antibody-antigen interactions, enzyme-substrate interactions, formation of duplexes or triplexes among polynucleotides and/or oligonucleotides, receptor-ligand interactions, and the like. As used herein, "contact" in reference to specificity or specific binding means two molecules are close enough that weak noncovalent chemical interactions, such as Van der Waal forces, hydrogen bonding, base-stacking interactions, ionic and hydrophobic interactions, and the like, dominate the interaction of the molecules.

"Uniform" in reference to spacing or distribution means that a spacing between objects, such as sequence markers, or events may be approximated by an exponential random variable, e.g. Ross, Introduction to Probability Models, 7^{th} edition (Academic Press, New York, 2000). In regard to spacing of sequence markers in a mammalian genome, it is understood that there are significant regions of repetitive sequence DNA in which a random sequence model of the genomic DNA does not hold. "Uniform" in reference to spacing of sequence markers preferably refers to spacing in uniques sequence regions, i.e. non-repetitive sequence regions, of a genome.

"Vector" or "cloning vector" refers to an extrachromosomal genetic element which can be used to replicate a DNA fragment in a host organism. A wide variety of cloning vectors are commercially available for use with the invention, e.g. New England Biolabs (Beverely, Mass.); Stratagene Cloning Systems (La Jolla, Calif.); Clontech Laboratories (Palo Alto, Calif.); and the like. Usually, cloning vectors used with the invention are bacterial plasmids.

### DETAILED DESCRIPTION OF THE INVENTION

In one aspect, the invention provides a method of enriching a pool of nucleic acid duplexes for heteroduplexes containing sequences that vary from those of a reference DNA population. In accordance with the invention, such enrichment is carried out enzymatically using one or more enzymes collectively having a mismatch recognition activity, a exonuclease activity, and a nucleic acid polymerase activity. In one aspect, these activities are provided in vivo by a mismatch repair system of a host microorganism, such as a bacteria. In another aspect, these activities are provided in vitro using commercially available reagents. In accordance with the invention, a mixture of test sequences, that is, nucleic acids suspected of containing one or more mutations or rare alleles, and corresponding reference sequences are denatured and reassociated so that duplexes form comprising heteroduplexes and homoduplexes. Whenever a subset of heteroduplexes contains mismatched sequences, the mismatches are corrected so that the sequences of strands from the test population are preserved, or selected, or heteroduplexes lacking mismatches are destroyed so that heteroduplexes containing mismatches are enriched in the resulting mixture. The preserved or selected sequences may then be denatured and reassociated with additional sequences from the reference population to once again form duplexes comprising homoduplexes and/or heteroduplexes. Fig. 1 illustrates the general concept of the invention. Reference DNA (10) from a genetic locus or a nucleic acid sequence derived therefrom is mixed (14) with test DNA from an equivalent segment of DNA from the genetic locus in other individuals or tissues, after which the DNAs are denatured and allowed to reassociated to form duplexes (16). The reference DNA populations and test DNA populations may be derived from a variety of sources, including genomic DNA, expressed genes, messenger RNA, and the like, of either plant, animal, or microbial origin, and may be from individuals of the same or different species, or from individuals of similar or different geographical or ecological environments. In one aspect, the test DNA populations are from one or more individuals having a medical condition, susceptibility, or disease of interest, and the reference DNA populations are from one or more individuals free of such condition, susceptibility, or disease. In another aspect, reference DNA populations and test DNA populations may be derived from cells or tissues of the same individual, such as normal cells or tissues and tumor cells or tissue, respectively, from a cancer patient, in order to assess or measure somatic mutations in a cancer. The test and reference DNA populations may be derived from genomic DNA, which allows comparison of expressed as well as non-expressed DNA sequences, such as genetic regulatory elements. In one embodiment, the reference and test DNA populations are prepared by digesting source DNA, e.g. genomic DNA, with the same restriction endonuclease(s). The respective fragments may then be inserted into cloning vectors to form libraries for storing and manipulating the populations of DNA fragments. Exemplary cloning vectors include pUC19, pNEB193, M13mp, pBluescript II, and the like (New England Biolabs, Beverly, Mass.: Stratagene, La Jolla, Calif.). Preferably, reference and test populations of DNA are prepared by cloning in vectors in order to minimize the random introduction of spurious mutations. If PCR is employed in the preparation, preferably high fidelity DNA polymerases are employed, such as Pfu DNA polymerase, or the like, and/or the number of amplification cycles is minimized, e.g. is less than 20.

In one aspect, a reference DNA population may be formed by directionally cloning restriction fragments of the source DNA for the reference strands into a cloning vector using conventional protocols or commercial kits, e.g.pBluescript II, pCMV-Script, or pAD-GAL4 XR (Strategene, San Diego); U.S. patents 5,512,468; 5,681,726; Scharf et al, Science, 233: 1076-1078 (1986); Kaluz et al, Nucleic Acids Research, 20: 4369-4370 (1992); Rashtchian et al, Anal. Biochem., 194: 9-15 (1992). In one embodiment, such a cloning vector is constructed with the following elements adjacent to the site for accepting the fragments of the reference DNA population: first primer binding site, first restriction site, second restriction site, and second primer binding site. Likewise, a test DNA population is formed by directionally cloning restriction fragments of the source of test DNA into a similar cloning vector. Amplicons containing either test DNA or reference DNA may be produced from the libraries of fragments by PCR. PCR products may be purified after amplification by use of commercially available solid phase column purification systems, e.g. QIAquick PCR Purification kit (Qiagen GmbH, Hilden, Germany).

As noted in Fig. 1, duplexes (16) of the reaction include perfectly matched homoduplexes of reference DNA and test DNA, perfectly matched heteroduplexes each comprising one strand of reference DNA and one strand of test DNA, and mismatched heteroduplexes each comprising one strand of reference DNA and one strand of test DNA wherein the strands are not exact complements of each other so that there are one or more mismatches (18), deletions (19), or insertions (20) in the test strand (denoted by "T") relative to the reference strand (denoted by "R"). In accordance with one aspect of the invention, mismatched heteroduplexes are enzymatically treated to recover and amplify the test DNA strands of such duplexes (22). One or more cycles (24) of such steps of duplex formation and treatment may be employed. In one aspect, a plurality of such cycles are employed to enrich the pool of DNA at step (24) with strands of test DNA sequences that differ from the reference DNA sequences. Preferably, the plurality of cycles is in the range of from 2 to 10; and more preferably, in the range of from 2 to 5. A predetermined number of cycles may be implemented, e.g. 2, or 3, or the like, or test strands may be labeled, e.g. with a fluorescent dye, and cycles may be continued until a predetermined level of fluorescence is measured, e.g. using a conventional fluorimeter.

Recovered strands (22) may be used in an additional cycle of selection. Strands (22) may be amplified and combined (26) with additional reference DNA to form another pool of DNA that is denatured and reassociated to form another mixture of duplexes (16).

After the desired level of enrichment is achieved, the mixture, or pool, of enriched DNA is sequenced (28) by a conventional technique, which may include Sanger sequencing, pyrosequencing, or resequencing using microarray technology. Sanger sequencing may be carried out on commercially available DNA sequencer, e.g. Applied Biosystems model 3700 (Foster City, CA), or the like. Pyrosequencing may be carried out on a commercially available instrument, e.g. Biotage model PSQ HS 96 (Kungsgatan, Sweden). Resequencing microarrays and their operation are disclosed in the following references that are incorporated by reference: Kennedy et al, Nature Biotechnology, 23: 1233-1237 (2003); Chee et al, Science, 274: 610-614 (1996); Wang et al, Science, 280: 1077-1082 (1998); Dong et al, Genome Research, 11: 1418-1424 (2001); Warrington et al, Human Mutation, 19: 402-409 (2002).

In any of the above sequencing techniques, readouts from the methods or instruments implementing the method are processed to produce base-call signals indicative of nucleotides in a target nucleic acid. As used herein, a "base-call signal" is the output of a data processing algorithm that takes raw data, or a readout signal, and makes a determination of the identity of a nucleotide, or nucleotides, at a particular location in a nucleotide sequence. Such determinations may include confidence measures that the determination is correct. An extensive literature is available for guidance in using such data processing algorithms to analyze electropherograms in Sanger sequencing to give estimates of allele frequencies. Exemplary references include the following, which are incorporated by reference: Amos et al, Am. J. Hum. Genet., 66: 1689-1692 (2000); Ewing et al, Genome Research, 8: 175-185 (1998); Ewing et al, Genome Research, 8: 186-194 (1998); Shaw et al, Genome Research, 8: 111-123 (1998); U.S. patents 6,048,689; 6,681,186; 6,274,317; 5,580,728; 5,853,979; 5,916,747; and the like. Likewise, an extensive literature is available for guidance in using microarrays, e.g. Affymetrix GeneChips™ (Santa Clara, CA), or like products, to detect variant sequences in a pool of DNA. Exemplary references include the following, which are incorporated by reference: Hacia et al, Nature Genetics, 14: 441-447 (1996); Warrington et al, Hum. Mut., 19: 402-409 (2002); Dong et al, Genome Research, 11: 1418-1424 (2001); Wang et al, Science, 280: 1077-1082 (1998); Cutler et al, Genome Research, 11: 1913-1925 (2001); Liu et al, Bioinfromatics, 19: 2397-2403 (2003); U.S. patents 6,342,355; 6,284,460; and the like. It would be within the ken of one of ordinary skill in the art to use currently available algorithms for generating base-call signals with the present invention to assess increases in base-call signals after one or more cycles of enrichment of rare alleles or mutations in a test sample. For example, in Sanger sequencing electropherograms such a base-call signal may appear as the increase in relative size of one of two peaks at a particular location in an electropherogram.

### Formation of Duplexes and Recognition of Mismatches

In another aspect of the invention, heteroduplexes are formed between single stranded DNA of the test DNA population and substantially complementary single stranded DNA of the reference DNA population. Such single stranded DNA may be produced in a variety of ways, including denaturation by heating, treatment with a chaotropic solvents, and the like, using techniques well known in the art, e.g. Britten et al. Methods in Enzymology, 29: 363-418 (1974): Wetmur et al, J. Mol. Biol., 31: 349-370 (1968). Preferably only complementary single stranded DNA from one population is selected for hybridization with the single stranded DNA of the other population, so that there is no possibility of forming nonproductive homoduplexes. Such stand isolation can be carried out in several ways, including asymmetric PCR, PCR with one nuclease-resistant primer followed by exonuclease digestion, melting the complements from avidin-captured biotinylated strands, and the like, e.g. Birren et al, editors, Genome Analysis: A Laboratory Manual, Vol. 1 (Cold Spring Harbor Laboratory Press, New York, 1997); Hultman et al, Nucleic Acids Research, 17: 4937-4946 (1989); Straus et al, BioTechniques, 10: 376-384 (1991); Nikiforow et al. PCR Methods and Applications, 3: 285-291 (1994); and the like, which references are incorporated by reference. Preferably, single stranded DNA from the respective populations is prepared by PCR with a nuclease-resistant primer followed by exonuclease digestion.

Whenever the method of the invention is applied to populations of DNA that include all or a substantial fraction of complete genomes, particularly mammalian or higher plant genomes, the step of forming duplexes preferably includes a step of forming subpopulations of DNA in order to reduce the complexity of the DNA populations prior to hybridization. As is well known in the art, e.g. Britten et al (cited above), the rate of a hybridization reaction depends on the complexity of the mixture of hybridizing nucleic acid strands. Thus, in order to reduce the time of forming heteroduplexes, the complexities of either one or both the reference DNA and the test DNA populations are preferably reduced prior to carrying out hybridization reactions for forming heteroduplexes. Preferably, complexities are reduced by partitioning the respective populations into subpopulations in well known ways, e.g. differential PCR amplification using sets of primers having different 3'-terminal nucleotides, e.g Pardee et al. U.S. Pat. No. 5,262,311: amplification after ligation of indexing linkers, e.g. Kato, U.S. Pat. No. 5,707,807: Deugau et al, U.S. Pat. No. 5,508,169; and Sibson, U.S. Pat. No. 5,728,524; and the like, which references are incorporated by reference. In one aspect of the invention, complexities are reduced enough to permit reassociation of ninety percent of the complementary single stranded DNA in 72 hours or less. In another aspect, complexity is reduced to permit reassociation of ninety percent of the complementary single stranded DNA in 48 hours or less, and in still another aspect, complexity is reduced so that reassociation of ninety percent of the complementary single stranded DNA takes place in 16 hours or less.

In embodiments wherein complex genomes, such as human genomes, are compared, a reference DNA population may consist of restriction fragments from an insert of a cloning vector, such as a phage, cosmid, BAC, PAC, YAC, or like system for storing and maintaining large genomic fragments. In one embodiment, a reference DNA population consists of restriction fragments from a BAC insert. Techniques for construction and manipulation of such cloning vectors is described in Birren et al, editors. Genome Analysis: A Laboratory Manual, Vol. 1, "Analyzing DNA," (Cold Spring Harbor Laboratory Press, New York, 1997); or like references. Preferably, restriction fragments making up a reference DNA population and/or a test DNA population have an average length in the range of several tens of basepairs, e.g. 50, to a few thousand basepairs, e.g. 2000, so that they may be easily cloned in readily available vectors and/or amplified by PCR. In another aspect, such restriction fragments have an average length in the range of 50 to 500 basepairs. In a further aspect, a reference DNA population derived from a BAC insert consists of from several hundred, e.g, two hundred, to several thousand, e.g, two thousand, fragments.

Once heteroduplexes are formed in accordance with the invention, heteroduplexes with mismatches may be distinguished from those forming perfectly matched duplexes in a manner which permits isolation of a selected strand of each of the mismatched heteroduplexes. Preferably, such selected strand is from the strand from the test DNA population. This may be accomplished with application of so-called chemical mismatch recognition techniques as well as enzymatically based mismatch recognition techniques, such as disclosed widely in the mutation detection literature, e.g. Taylor (cited above), Cotton (cited above); Cotton, U.S. Pat. No. 5,698,400; Modrich et al, U.S. Pat. No. 5,702,894; Ganguly et al, Genomics, 4: 530-538 (1989); Ganguly et al, Nucleic Acids Research, 18: 3933-3939 (1990); Lu et al, Genomics, 14:249-255 (1992); Ellis et al, Nucleic Acids Research, 22: 2710-2711 (1994); Maniatis et al, U.S. Pat. No. 4,946,773; Smooker et al. Mutation Research, 288: 65-77 (1993): and the like, which references are incorporated by reference.

In one aspect, mismatched heteroduplexes are distinguished from perfectly matched heteroduplexes and homoduplexes by one or more enzymes that recognize(s), or is (are) affected by, mismatched sequences. Such enzymes include resolvases. RNase As, methyl-directed mismatch repair enzymes, mutY proteins, cel I, exonucleases, and the like. In another aspect, mismatched heteroduplexes are recognized by a single stranded exonuclease which requires a double stranded DNA substrate. That is, the exonuclease binds to a double stranded DNA substrate, but digests only one of the two strands of the substrate. When such an exonuclease encounters a mismatched duplex, which disrupts the substrate structure, digestion is significantly inhibited, or stops altogether. An example of such an exonuclease is exonuclease III from Escherichia coli, which is described fully in Rogers et al. Methods in Enzymology, 65: 201-211 (1980); and Guo et al, Methods in Enzymology, 100: 60-97 (1983) (which references are incorporated by reference). Exonuclease III is a standard reagent of molecular biology and is widely available commercially, e.g. New England Biolabs (Beverly, Mass.).

### Example 1

### In Vitro Recovery of Test DNA Strands From Mismatched Heteroduplexes

In this example, an in vitro selection and amplification procedure is applied to enrich a pooled sample of DNA for variants that differ from a reference DNA population. Mismatched heteroduplexes are selected and test DNA strands recovered by using MutS protein to protect mismatches from digestion by a 3'→ 5' exonuclease. The use of MutS together with the exonuclease activity of T7 polymerase in such protection assays is described in Ellis et al, Nucleics Acid Research, 22: 2710-2711 (1994), which is incorporated by reference. As shown in Fig. 2A, amplicons (200) and (202) are produced from vectors containing reference DNA fragments and test DNA fragments, respectively, and the following elements. Each amplicon contains restriction site (204) used (in one embodiment) to insert the respective fragment into the vector. Each amplicon also contains a segment, (208) in the reference amplicon and (210) in the test amplicon, which is disposed between the first primer binding site (206) and restriction site (204). Sequences of segment (208) and (210) are identical, except for a nucleotide difference at a single position, selected so that a single mismatch forms when duplexes are formed between the two segments, as illustrated by (226) and (230). As illustrated below, the duplex between segments (208) and (210) that is formed after treatment with an exonuclease keeps the strands together (so that the full heteroduplex can be reconstituted) whenever the test and reference DNA fragments contain a mismatch.

Returning to Fig. 2A, selected single strands of the amplicons are produced as described by Straus et al, BioTechniques 10: 376-384 (1991), which reference is incorporated by reference. Primers (232) and (234) are cross-hatched to indicate that they contain exonuclease resistant linkages. As a result, the 5' end of strand (211)(the "top" strand) of amplicon (212) is resistant to 5'→ 3' exonuclease digestion. Likewise, the 5' end of strand (213) (the "bottom" strand) of amplicon (214) is resistant to 5'→ 3' exonuclease digestion. Many different nuclease-resistant nucleotides may be employed where called for in the invention provided that such moieties are compatible with PCR conditions. For example, Eckstein, editor, Oligonucleotides and Analogues: A Practical Approach (IRL Press, Oxford, 1991); Mesmaeker et al, Current Opinion in Structural Biology, 5: 343-355 (1995); and like references, describe several nuclease-resistant oligonucleotides. In one aspect, phosphorothioate linkages between nucleosides may be used to confer nuclease resistance. After digestion (216) and (218) with a 5'→ 3' exonuclease, e.g. T7 gene 6 exonuclease (available from United States Biochemical), single stranded fragments (217) and (219), respectively, are produced.

Fragments (217) and (219) are mixed under duplex-forming conditions (220) so that either mismatched heteroduplexes (222) form (with respect to the test and reference DNA fragments) or perfectly matched heteroduplexes (224) form. The duplexes derived from segments (208) and (210) will always have at least one mismatch (226) or (230), whether such duplex is in mismatched heteroduplex (222) or in perfectly matched heteroduplex (224).

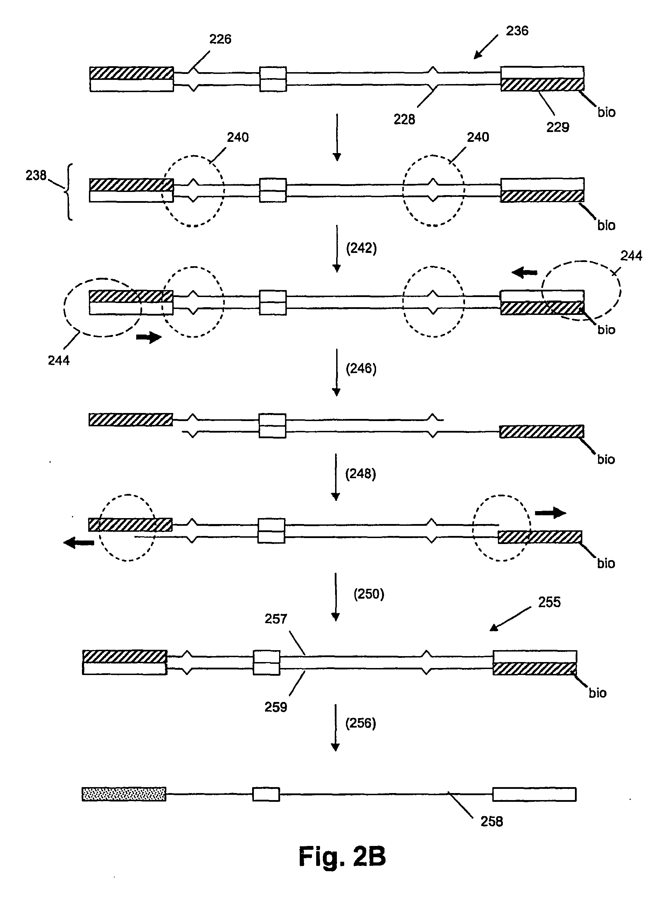

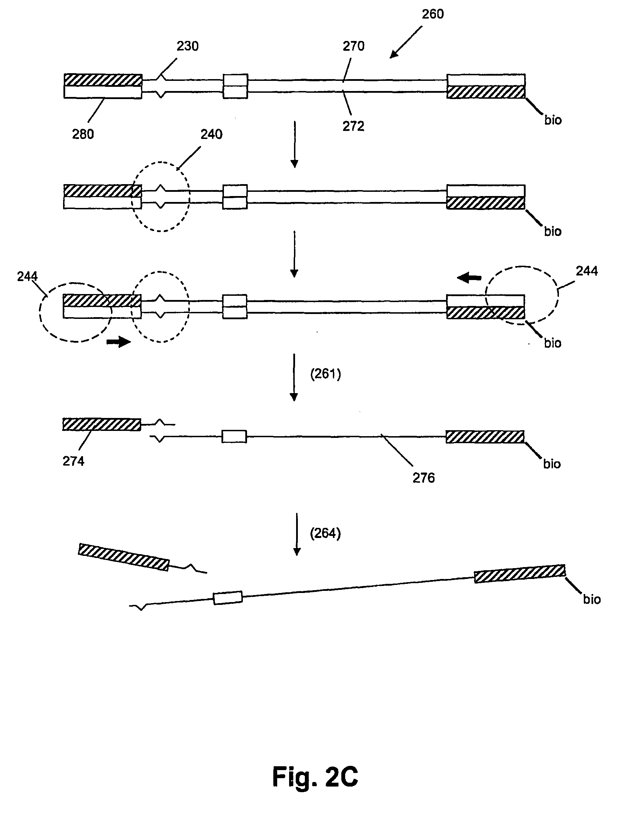

Figs. 2B and 2C illustrate how the mismatches are employed to recover strands only from mismatched heteroduplexes using MutS protection. In Fig. 2B, mismatched heteroduplex (236) (having primer (229) with a capture moiety, such as biotin) is treated with MutS protein so that complex (238) forms comprising MutS proteins bound to mismatches (226) and (228). Following Ellis et al (cited above), complex (238) is then treated (242) with a 3'→ 5' exonuclease (244), such as T7 polymerase, which digests the 3' ends of mismatched heteroduplex (236). Digestion continues (246) until the 3'→ 5' exonuclease reaches a mismatch protected by a MutS protein. At this point, e.g. 3-5 minutes after addition of the exonuclease, the reaction may be stopped by adding a chelator, such as EDTA. Alternatively, nucleoside triphosphates may be added so that mismatched heteroduplex (236) is reconstituted (248) by the T7 polymerase. Reconstituted mismatched heteroduplex (255) is then isolated with a solid phase support, e.g. avidinated magnetic beads (Dynal, Norway), reference strand (257) is melted off, and copies (258) of test strand (259) are made. As illustrated, whenever a mismatch occurs in the part of the heteroduplex comprising the test and reference DNAs, there will be a portion of the heteroduplex that will not be digested. This portion contains at least the segments (208) and (210). Accordingly, the length and composition of segments (208) and (210) are selected so that a duplex is maintained under the reaction conditions. In one aspect, segments (208) and (210) each have a length in the range of from 20 to 40 nucleotides and are composed of from forty to seventy-five percent G's or C's.

In Fig. 2C, heteroduplex (260) is illustrated that has a perfectly matched duplex between reference fragment (270) and test fragment (272). As above, MutS (240) binds to mismatch (230) and the resulting complex is treated with a 3'→ 5' exonuclease (244), such as T7 polymerase, which digests (261) the 3' ends of heteroduplex (260) to give fragments (274) and (276) that dissociate (264). Although fragment (276) is capture by the same solid phase as the full-length strand above, no copies will be generated because primer binding site (280) is lost.

### Example 2

### In Vivo Recovery of Test DNA Strands From Mismatched Heteroduplexes with a Methyl-Directed Mismatch Repair System

In this example, an in vivo selection and amplification procedure is applied to enrich a pooled sample of DNA for variants that differ from a reference DNA population. Mismatched heteroduplexes are selected and test DNA strands recovered by using a bacterial methyl-directed mismatch repair system that has been coupled to a marker that is co-repaired with mismatches in duplexes between a reference DNA strand and a test DNA strand. The in vivo enrichment employs the mismatch repair detection (MRD) techniques disclosed in U.S. patents 6,406,847 and 6,709,827; U.S. patent publication 2003/0003472; and reference Fakhrai-Rad et al, Genome Research, 14: 1404-1412 (2004); which patents, publication, and reference are incorporated by reference. Briefly, as is illustrated in Fig. 3, the MRD selection process comprises three steps. Test DNA population (300) comprising fragments, e.g. (306) and the like, labeled 1 through K, are combined under duplex-forming conditions with methylated reference DNA population (302) comprising single stranded reference DNA fragments in vectors (303) and with unmethylated open vectors (304) comprising marker element that is inactivated by the presence of insertion (305). In one embodiment, the marker is a Cre recombinase gene that when repaired inactivates a lox-bracketed element of an F1 plasmid which, in turn, permits the host to grow in a one selective medium. Without inactivation, the host grows on another selective medium. The host cell have a methyl-directed mismatch repair (MMR) system that is activated by a mismatch in a duplex test DNA (300) and reference DNA (302). Upon activation, the MMR system repairs both this latter mismatch and the inactivating insertion in the marker gene. The annealed components (300), (302), and (304) form (308) double stranded circles (310), which are ligated and transformed (312) into host cells (314). Host cells (314) are then plated onto two different selective media that activate markers either indicating variation (316) in the test DNA with respect to the reference DNA or no variation (318).

94 tumor samples were utilized that had already been sequenced for all the coding exons of BRCA1 gene. There were 10 known SNPs in the BRCA1 exons. To assess sensitivity at different allele frequency levels genomic pools were constructed to test each of these SNPs at frequencies ranging from 1-30%. 95 amplicons were designed that encompass all the exons of BRCA1 and BRCA2 genes. Homozygous DNA was used from hydratidiform mole as a template for PCR. The PCR products were pooled and cloned *en masse* to generate the reference DNA. 5 different pools were constructed. The first pool was an equimolar ratio of all 94 samples. The other 4 pools were constructed using 5 genomic samples and the homozygous mole DNA. The five genomic samples were very carefully quantitated and mixed in equal amounts. This DNA pool, the 5-genome pool, were again carefully quantitated and mixed with 4 different ratios of excess mole DNA. The 4 pools had 1 part of the 5-genome pool to 6, 13, 34, or 69 parts of the mole DNA. The mole DNA obviously had no variation to itself and so it effectively acted as a diluent of variant alleles in the other samples. The frequency of an allele in a pool was then the frequency of that allele among the 5 individuals divided by the dilution factor. For example an allele that is present in 7/10 chromosomes among the 5 individuals has the final frequency of 10%, 5%, 2%, and 1% in the four pools.

These 4 pools were used as well as the pool of all 94 samples as a template for 95 PCR reactions amplifying BRCA1 and BRCA2. The 95 PCR reactions from each of the 5 genomic mixtures were pooled and subjected to two rounds of MRD enrichment. Eighty nine out of ninety five fragments yielded sequencing results from at least one of the 5 MRD reactions and the failure in 4/6 cases was in the initial genomic PCR reaction. The sequencing traces from the standard and those from each MRD reaction were independently compared and SNPs were called. A list of the SNPs detected in each pool was then compiled and compared with the known SNPs.
False Negatives. Two of the 10 SNPs were not detected in any of the pools. One of these SNPs was on an amplicon that failed the initial PCR step. The other SNP was missed even when present at 30% frequency. Further investigation of the sequencing data showed that another SNP that is perfectly correlated with this missing SNP was present one nucleotide away from the 3' end of one of the primers. This secondary SNP caused allele specific amplification resulting in the absence of the primary SNP from the amplicon. Given that these two non-detected SNPs (one for amplification failure and the other because of allelic drop-out due to secondary SNPs) would be missed in all PCR-based methods we did not include them in the further analysis.
Sensitivity. As can be seen in Figure 4A all the variants were detected when their frequency was at least 10%. The discovery rate decreases when the allele frequency is lower with most the variants still detected when present at 1% frequency. An example of a SNP detected at the 1% frequency is shown in Figure 5. Variant 8 is in the same amplicon as variant 7 and is not detected underscoring the difficulty with this technology to detect two SNPs with different frequencies on the same amplicon.
False Positives. In addition to the already known SNPs, we found 8 new variants (7 of them in BRCA2 and 1 in BRCA1) in the 4 pools constructed from pooling the 5 individuals. We sequenced these 8 amplicons in the 5 individuals that made up the pools and detected 7/8 variants in at least one individual. The frequency of each SNP in the initial genomic mixture was calculated and is depicted in Figure 4B. As seen in Figure 4B, the detection of SNPs at 1% frequency is robust and some variants were detected at a frequency as low as 0.5%.

The last SNP that was detected in one of the pools could not be seen in any of the individuals. This estimates that this method generates a false positive in about 0.25% of the amplicons (1 false positive in almost 400 amplicons in the 4 pools) and that the proportion of false positive SNPs is about 2% of all SNPs detected (in the 4 pools). It is likely that this false positive is secondary to PCR-induced mutation that happened to be unusually enriched in one of the 4 pools. Of note is that this false positive variation had a very modest enrichment and one could potentially reduce the false positive results by implementing more rigorous rules about the extent of enrichment for accepting a variation. Given the traces seen in this study, such rules would likely decrease the sensitivity of detecting alleles at <1% frequency but have minimal effect for alleles that are >1% that are generally robustly enriched.
Reproducibility. As a first indication of reproducibility we noted that variation number 5 was present in another overlapping amplicons. This variation was detected in all 5 pools in the two amplicons (data not shown). To further assess reproducibility we did the MRD enrichment in duplicates for the 4 pools that were constructed from 5-genome pool and the mole DNA. The results were essentially identical for all the duplicates with two exceptions: missing one variation at 0.7% frequency and the absence of the false positive SNP that was described above. So all the variations with an allele frequency of 1% or higher that were detected in the first experiment, were also detected in the replication experiment. This underscores the general reproducibility of these results as well as the fact that the false positive result is due to random rather than systematic error consistent with an enrichment of a PCR-induced mutation.
Extent of enrichment. We have estimated from the sequencing traces the frequency of the SNPs in the variant pool as described previously, Kwok et al, Genomics, 23: 138-144 (1994). Since the starting frequency of each SNP was known we were able to estimate the level of enrichment for each SNP. Fig. 5 shows the average level of enrichment for variants at different allele frequencies. Rarer alleles have higher fold enrichment than common alleles. The incomplete enrichment stems from the background of the system that is mainly a reflection of PCR error as described in the discussion section.

### Construction of reference DNAs

All enzymes used in this example were from New England Biolabs (NEB) unless otherwise specified. Amplicons were designed to amplify exons and flanking intron sequences. Primer selection was done through a batch version of *PRIMER3* (Fakhrai-Rad et al, cited above). The amplicon sizes were designed to have approximately the same size 100-500 base pairs. Restriction sites were added to the 5' ends of all the primers; the sites were Cla I and Sac II sites for the chromosome 21 study and Cla I and Asc I for the BRCA study. PCR was performed using 0.8 units of *pfu* turbo hotstart (or pfu ultra in the BRCA experiment) polymerase (Stratagene) with the standard buffer, dNTP concentration in 20 µl reaction and a human-mouse hybrid containing one copy of chromosome 21 (Corriell) as a template or hydratidiform mole (Corriell) for the BRCA study. The cycling conditions were 95 °C 30", 57 °C 1', and 72 °C 1" for 35 cycles. The PCR products were run on a 1% agarose gel and equal mass were pooled and purified through gel electrophoresis. The product pool was digested with C1a I and Sac II (or Cla I and Asc I) @ 37 °C for 2 hours. One hundred ng of the digested products were ligated to 100 ng of digested and dephosphorylated plasmid pMRD300 carrying the active Cre gene. After overnight ligation at 16 °C, a QiaQuick purification column (Qiagen) was performed to purify the product from the ligase and salts. Transformation is then done to a standard cloning strain like DH5α and selection for transformants was done in liquid by adding 100µg/ml Carbenicillin. DNA was prepared and transformed into GM2929. The two step transformation is because dam strains have low efficiency of transformation. DNA obtained from this transformation was used in later steps.

### MRD protocol

For the BRCA study, different pools were generated. In one pool equal amounts of each of 94 samples were mixed. In the other 4 pools, 5 genomic DNA samples were mixed after picogreen and real time PCR quantitation. The pool along with DNA from a hydratidiform mole were carefully quantitated again using picogreen and real time PCR. The pool and the mole DNA were then mixed at 4 different ratios.

PCR reactions using the genomic pool as a template was performed using *pfu* turbo hotstart (or pfu utra for the BRCA experiment) polymerase using a similar protocol as described above. The PCR products from each population were pooled and a purification column QiaQuick (Qiagen) was performed. Methylation of the PCR products was carried out by addition of Tris PH=7.6 to a final concentration of 50 mM as well as SAM (NEB) to a final concentration of 80 µM and 8 units of dam methylase (NEB) at 37°C for 1or 2 hrs. The PCR pool was then digested with Cla I and Sac II at 37°C for 1-2 hrs.

For each MRD reaction, 2 µg of the above PCR product pool was mixed with 2 µg of the pool of the unmethylated standard DNA and 2 µg of digested vector carrying the inactive Cre gene pMRD400. pMRD400 is the same as pMRD300 except for a 5 bp deletion in the Cre gene. The three components were concentrated to 10 µl using a QiaQuick minielute column (Qiagen). 0.5 µl of 0.5M EDTA 0.5 µl of 200 mM Tris PH=7.6, 0.5 µl 20XSSC, and 1.25 µl of freshly diluted 1M NaOH was added and incubation for 15 min at room temperature followed. 1.25 µl of 2M Tris PH=7.2 and 12.5 µl formamide were then added and reannealing was allowed to occur at 42°C overnight. The hybridization mixture was desalted using a column (Edge Biosystems). Three µl Taq Ligase buffer and 5 units Mbo I was added and incubated for 15 minutes followed by addition of 40 units Taq Ligase (NEB) and further incubation followed at 65 °C for 30 min. Fifty units of Exonuclease III (USB) and 20 units of T7 Exonuclease (USB) were added and incubated at 37 °C for 30 min. Ten µl of SOPE Resin (Edge Biosystems) was added to eliminate single stranded DNA and a QiaQuick cleanup (Qiagen) was done before transformation.

Transformation of the MS3 strain was done by electroporation (Micropulser, BioRad, USA). The electrocompetent MS3 cells preparation as well as the electroporation procedure was done as recommended, Ausubel et al, Current Protocols in Molecular Biology (John Wiley & Sons, New York, 1999). During the 1 hour recovery phase 1 µl of 0.1M IPTG was added into 1 ml SOC medium (Invitrogen). The culture was plated onto a plate supplemented with Carbenicillin (75 µg/ml), and Tetracycline (3.25 µg/ml) to select for colonies with variant alleles. The next day, all the colonies from the plate were collected into a tube with 1 ml of LB. DNA from the cells obtained after the overnight growth with the selective media was minipreped using the QIAPrep columns (Qiagen) as recommended by the manufacturer.

### Sanger Sequencing and sequence analysis

Using DNA from the variant pool of the two electroporations, we performed PCR reactions followed by bidirectional sequencing for each amplicon. For the chromosome 21. project the sequencing traces were analyzed using Sequencher software and compared to the genome database using BLAT (http://genome.ucsc.edu/cgi-bin/hgBlat?command=start). SNPs were called when the dominant base in the enriched pool was different from the hybrid or when mixed peaks were seen in both strands of the enriched pool. For the BRCA study Mutation Surveyor software (SoftGenetics) was used. SNPs automatically called in both strands were accepted directly. Variations called in only one strand were then inspected manually.

## Claims

1. A method of identifying a rare allele at a genetic locus in a population of individuals, the method comprising the steps of:
(a) forming duplexes between test sequences and reference sequences thereof such that heteroduplexes containing one or more mismatches form whenever a rare allele is present in a test sequence, and such that the test sequences initially are sequences from the genetic locus of individuals of the population;
(b) enriching the amount of heteroduplexes containing such one or more mismatches by treating the duplexes with enzymes having mismatch-recognition activity, nucleic acid polymerase activity, and exonuclease activity, the mismatch-recognition activity binding to each of the one or more mismatches, the exonuclease activity digesting one or more portions of each heteroduplex containing such one or more mismatches, and the nucleic acid polymerase activity replacing such digested portions whenever a heteroduplex contains at least one mismatch between a test sequence and a reference sequence;
(c) repeating steps (a) and (b) until at least five percent of the duplexes are heteroduplexes containing one or more mismatches or until steps (a) and (b) have been performed a predetermined number of times; and
(d) sequencing at least one strand of the duplexes to form a plurality of base-call signals, the plurality of base-call signals indicating the identity and quantity of nucleotides at each sequence position of a strand of the duplex.

2. The method of claim 1 wherein said step of enriching is carried out in vitro.

3. The method of claim 1 or claim 2 wherein said predetermined number is between 1 and 3.

4. The method of any one of claims 1 to 3 wherein said step of sequencing is carried out with Sanger sequencing or pyrosequencing, or with a resequencing microarray.

5. The method of claim 1 wherein said step of enriching is carried out in vivo using a methyl-directed mismatch repair system.

6. The method of claim 5 wherein said reference sequences are from normal tissue of a patient and said test sequences are from a disease tissue of the patient.

7. The method of claim 5 or 6 wherein said predetermined number is between 1 and 3.

8. The method of any one of claims 5 to 7 wherein said step of sequencing is carried out with Sanger sequencing or pyrosequencing, or with a resequencing microarray.

9. Use of one or more enzymes collectively having mismatch recognition activity, exonuclease activity and nucleic acid polymerase activity for enriching a pool of nucleic acid duplexes between test sequences and references sequences for mismatch duplexes, optionally for identifying a rare allele at a genetic locus in a population of individuals.

10. Use as claimed in claim 9, wherein the mismatch recognition activity allows enzyme binding to mismatch duplexes, the exonuclease activity allows digestion of portions of mismatched duplexes, and wherein the nucleic acid polymerase activity allows replacement of digested portions whenever a duplex contains at least one mismatch between a test sequence and a reference sequence.
